(19) Europäisches Patentamt
European Patent Office
Office européen des brevets

(11) Numéro de publication: **0 105 008**
**B1**

# (12) FASCICULE DE BREVET EUROPEEN

(45) Date de publication du fascicule du brevet: **02.04.86**

(51) Int. Cl.[4]: **C 07 C 76/06,** C 07 C 79/24, C 07 C 79/46, C 07 C 143/78

(21) Numéro de dépôt: **83420140.2**

(22) Date de dépôt: **11.08.83**

(54) **Procédé d'élimination d'agents nitrosants de composés aromatiques nitrés.**

(30) Priorité: **07.09.82 FR 8215319**

(43) Date de publication de la demande: **04.04.84 Bulletin 84/14**

(45) Mention de la délivrance du brevet: **02.04.86 Bulletin 86/14**

(84) Etats contractants désignés: **AT BE CH DE FR GB IT LI LU NL SE**

(56) Documents cités:
**DE - B - 1 276 049**
**DE - C - 885 546**
**FR - A - 790 561**
**FR - A - 1 551 106**
**GB - A - 1 100 494**
**US - A - 2 194 923**
**US - A - 2 796 446**

**Le dossier contient des informations techniques présentées postérieurement au dépôt de la demande et ne figurant pas dans le présent fascicule.**

(73) Titulaire: **RHONE-POULENC AGROCHIMIE, 14-20, rue Pierre Baizet, F-69009 Lyon (FR)**

(72) Inventeur: **Muller, Roger, 30 rue Soeur Bouvier, F-69005 Lyon (FR)**
Inventeur: **Ratton, Serge, Vaulx Milieu, F-38290 Verpilliere (FR)**
Inventeur: **Allandrieu, Christian, 19 rue Clément Michut, F-69100 Villeurbanne (FR)**

(74) Mandataire: **Chrétien, François et al, RHONE-POULENC AGROCHIMIE BP 9163, F-69263 Lyon Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de traitement de dérivés aromatiques nitrés pour éliminer les agents nitrosants qu'ils contiennent.

Ces agents nitrosants résultant de l'étape de nitration de dérivés aromatiques correspondants, et constitués essentiellement d'acide nitreux, sont des impuretés gênantes des dérivés aromatiques nitrés, produits finis ou intermédiaires dans leurs diverses applications, notamment les explosifs, les produits pharmaceutiques et les produits agrochimiques.

Certains procédés ont été décrits pour l'élimination d'agents nitrosants de dérivés benzéniques nitrés et notamment le chloro-1, dinitro-2,6 trifluorométhyl-4 benzène intermédiaire de l'herbicide connu sous le nom de trifluraline, en particulier par traitement à l'eau, sous pression au moins partielle de vapeur d'eau, ou encore à chaud en présence d'une base minérale. Si ces traitements se montrent efficaces dans le cas des produits cités, leur efficacité est diminuée lorsqu'on les applique à des nitrophénols ou à des acides benzoïques nitrés, tant en ce qui concerne l'élimination des agents nitrosants que la pureté des produits obtenus et le rendement en produit purifié.

L'invention a pour but de fournir un procédé efficace d'élimination des agents nitrosants dans ces dérivés benzéniques nitrés, et ce dans des conditions économiquement acceptables.

Elle concerne plus particulièrement un procédé d'élimination d'agents nitrosants de dérivés aromatiques nitrés, caractérisé en ce qu'on traite un produit brut constitué essentiellement d'un composé de formule générale:

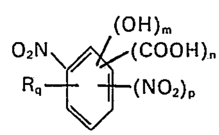
(I)

dans laquelle:

R, identique ou différent, représente un atome d'hydrogène ou d'halogène, un radical alcoyle contenant de 1 à 4 atomes de carbone, le groupe trifluorométhyle, sulfonamido ou méthylsulfonyle, et

m, n, p, q représentent chacun un nombre entier égal à 0 ou 1, q pouvant de plus être égal à 2, par une base, en solution dans un solvant organique inerte.

Parmi les composés susceptibles d'être traités selon l'invention, des dérivés hydroxylés et/ou carboxylés sont préférés. On a notamment obtenu de bons résultats avec des nitrophénols substitués, en particulier les dinitrophénols de formule:

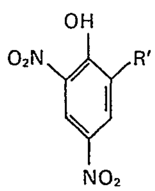
(II)

dans laquelle R' est un radical alcoyle contenant de 1 à 4 atomes de carbone, tels que le dinitroorthocrésol, le dinitroorthosec.-butylphénol ou dinosèbe et le dinitroorthotert.-butylphénol ou dinoterbe.

Au sens de la présente invention, on entend par produit brut un produit qui a été isolé après sa fabrication et qui est essentiellement débarrassé de l'acide nitrique résiduel provenant de la phase de nitration.

Le dérivé aromatique à purifier doit être, selon l'invention, traité en solution dans un solvant organique inerte, miscible ou non à l'eau. Comme solvant convenable, on peut citer des alcanols tels que ceux contenant de 1 à 4 atomes de carbone, notamment le méthanol et, de préférence, l'éthanol, mais aussi des phénols comme le phénol ou le xylol, ainsi que des éthers aliphatiques tels que l'éther de diéthyle et, de préférence, l'éther de diisopropyle, ou aromatiques tels que l'anisole (ou éther de méthyle et de phényle), ou encore hétérocyclique comme le tétrahydrofuranne, des polyéthers tels que les glymes, notamment le glyme, le diglyme ou le tétraglyme. On peut avoir également recours à des hydrocarbures aliphatiques tels que l'hexane, ou aromatiques tels que benzène, toluène ou xylène, et des hydrocarbures chlorés aliphatiques comme le dichloréthane, ou aromatiques comme les chlorobenzènes.

Le traitement devant se faire en milieu liquide, ces solvants sont utilisés en quantité suffisante pour dissoudre la totalité du dérivé aromatique nitré à purifier. Des quantités trop importantes sont à éviter pour ne pas alourdir la phase de séparation du produit purifié.

Le procédé selon l'invention nécessite la présence d'une base en quantité suffisante pour neutraliser les agents nitrosants. Celle-ci se situe entre 0,1 et 50%, et de préférence entre 0,1 et 30 et plus spécialement entre 1 et 25% en moles par rapport au dérivé aromatique nitré brut. La base peut être minérale, par exemple alcaline telle que soude, potasse, ammoniaque ou carbonate alcalin, telle quelle ou en solution, de préférence concentrée, ou organique telle qu'une amine primaire de préférence aliphatique, par exemple une alcoylamine, notamment de 1 à 6 atomes de carbone, comme la n-propylamine ou la n-butylamine ou une alcanolamine comme l'éthanolamine. On peut utiliser un mélange de deux bases distinctes et en particulier une base minérale avec une base organique.

La purification par la base en milieu solvant, selon l'invention, peut s'effectuer à une température comprise entre la température ambiante et la température de reflux du milieu réactionnel.

La durée de la réaction dépend de plusieurs paramètres tels que solvant, base, solubilité du dérivé aromatique nitré, etc. On a constaté que des temps allant de 15 min à plusieurs heures conviennent bien.

Une fois le traitement de purification terminé, le produit purifié est séparé par un procédé connu. Dans le cas d'un nitrophénol traité dans un solvant miscible à l'eau, on relargue par addition d'eau et on élimine le solvant par exemple par distillation

ou entraînement azéotropique, on refroidit et on filtre. Dans le cas d'un solvant non miscible à l'eau, on effectue un lavage de la solution organique, de préférence à chaud, puis le nitrophénol est cristallisé par refroidissement ou par évaporation du solvant.

Les produits purifiés par le traitement selon l'invention contiennent une quantité considérablement réduite d'agents nitrosants pouvant atteindre seulement quelques parties par million. De plus, ce résultat est obtenu avec un excellent rendement de purification associé à une grande pureté du produit final, ce qui permet une utilisation du procédé dans des conditions tout à fait compatibles avec une exploitation industrielle.

Les exemples suivants sont donnés pour illustrer les conditions de mise en œuvre et les résultats obtenus par le procédé selon l'invention.

Dans ces exemples, la teneur du produit (brut ou purifié) en agents nitrosants est exprimée en équivalent pondéral, en parties par million, en nitrite de soude par rapport au dérivé aromatique nitré traité. Le nitrite de soude a été choisi comme modèle d'agent nitrosant. On a ainsi pu, pour le dosage des agents nitrosants, utiliser une méthode adaptée de la méthode colorimétrique au réactif de Griess (cf. «Spot Tests in Organic Analysis», F. Feigl éd., 1966, p. 2921). Par ailleurs, la pureté du produit brut et du produit fini a été déterminée par chromatographie en phase gazeuse (CPG). Dans la suite, on entend par rendement de purification le rapport exprimé en pourcentage de la quantité de produit obtenu sur la quantité du produit engagé, les deux quantités étant exprimées en produit pur. De plus, on entend par rendement de récupération le rapport exprimé en pourcentage de la quantité de produit obtenu sur la quantité du produit engagé, les deux quantités étant exprimées en produit brut. Enfin, sauf indication contraire, les pourcentages sont exprimés en poids.

*Exemple 1:*

a) On dissout à froid 20 g de dinoterbe technique, à 81% en poids de dinitrotert.-butylphénol et contenant 1900 ppm d'agents nitrosants, dans 100 ml d'éthanol à l'ébullition. On ajoute 1 g de soude aqueuse à 30% et on chauffe pendant 3 h. Puis on relargue le produit par addition de 200 ml d'eau. Ensuite, on concentre le milieu aux deux tiers par distillation avec élimination de l'azéotrope éthanol/eau. On refroidit, on filtre et on lave sur filtre trois fois avec 50 ml d'eau.

Dans ces conditions, on constate, après analyse, que:
- la teneur en agents nitrosants est ramenée à 40 ppm,
- le dinoterbe obtenu a un titre de 93,5%,
- le rendement de récupération est de 83,0%,
- le rendement de purification est de 95,2%.

b) En opérant dans les mêmes conditions, mais en utilisant 2 g de soude à 30% et en ne chauffant que ½ h, on constate que:
- la teneur en agents nitrosants est ramenée à 30 ppm,
- le dinoterbe purifié a un titre de 97%,
- le rendement de récupération est de 79,5%,
- le rendement de purification est de 95,2%.

*Exemple 2:*

On opère comme à l'exemple 1b, si ce n'est que l'éthanol est remplacé par 200 ml de méthanol.

Dans ces conditions, on constate que:
- la teneur en agents nitrosants est ramenée à 40 ppm,
- le dinoterbe otenu a un titre de 97,0%,
- le rendement de récupération est de 76,5%,
- le rendement de purification est de 91,6%.

*Exemple 3:*

On opère comme à l'exemple 1b, en remplaçant l'éthanol par 100 ml de méthanol et la soude par 1,8 g d'ammoniaque à 28%. La durée de chauffage est de 2 h.

Dans ces conditions, on constate que:
- la teneur en agents nitrosants est ramenée à 40 ppm,
- le dinoterbe obtenu a un titre de 97,4%,
- le rendement de récupération est de 73,5%,
- le rendement de purification est de 88,4%.

*Exemple 4:*

a) On opère comme à l'exemple 1b, si ce n'est que la soude est remplacée par 0,01 g de n-butylamine.

Dans ces conditions, on constate que:
- la teneur en agents nitrosants est ramenée à 70 ppm,
- le dinoterbe a un titre de 90,3%,
- le rendement de récupération est de 87,5%,
- le rendement de purification est de 97,6%.

b) Si on répète l'expérience en ajoutant en plus 1 g de soude à 30%, on constate, après réaction, que:
- la teneur en agents nitrosants est ramenée à 40 ppm,
- le dinoterbe a un titre de 93,5%,
- le rendement de récupération est de 83%,
- le rendement de purification est de 95,8%.

*Exemple 5:*

On opère comme à l'exemple 1 en remplaçant l'éthanol par 800 ml de méthanol, et la base par 1 g d'éthanolamine. On laisse réagir pendant 3 h à température ordinaire.

Après la réaction, on évapore à sec le milieu et on reprend par 200 ml d'eau, on filtre, lave quatre fois avec 50 ml d'eau.

Dans ces conditions, on constate que:
- la teneur en agents nitrosants est ramenée à 20 ppm,
- le dinoterbe a un titre de 96,4%,
- le rendement de récupération est de 64%,
- le rendement de purification est de 76,2%.

*Exemple 6:*

a) On opère comme à l'exemple 1b, en remplaçant l'éthanol par 200 ml d'éther de diisopropyle. Après la réaction, on évapore le milieu aux trois quarts du volume, on laisse revenir à température

ambiante, on filtre et on lave trois fois avec 50 ml d'eau.

Dans ces conditions, on constate que:
- la teneur en agents nitrosants est ramenée à 15 ppm,
- le dinoterbe a un titre de 99,3%,
- le rendement de récupération est de 75%,
- le rendement de purification est de 91,9%.

b) On opère comme en a, si ce n'est que dans le post-traitement on effectue, avant l'évaporation du milieu aux trois quarts du volume, un lavage avec deux fois 25 ml d'eau chaude (70°C).

Dans ces conditions, on constate que:
- la teneur en agents nitrosants est ramenée à 8 ppm,
- le dinoterbe a un titre de 99,9%,
- le rendement de récupération est de 75,5%,
- le rendement de purification est de 93,1%.

*Exemple 7:*

On opère comme à l'exemple 1b, si ce n'est qu'on part d'un dinoterbe brut à 97,4% de dinitroorthotert.-butylphénol et contenant 140 ppm d'agents nitrosants; la quantité d'éthanol engagée est de 125 ml, la quantité de soude engagée est de 0,5 g.

Dans ces conditions, on constate que:
- la teneur en agents nitrosants est ramenée à 3 ppm,
- le dinoterbe purifié a un titre de 99,9%,
- le rendement de récupération est de 94,5%,
- le rendement de purification est de 96,9%.

## Revendications

1. Procédé d'élimination d'agents nitrosants dans des dérivés aromatiques nitrés, caractérisé en ce qu'on traite un produit brut qui a été isolé après sa fabrication et substantiellement débarrassé de l'acide nitrique résiduel provenant de la phase de nitration constitué essentiellement d'un composé de formule générale:

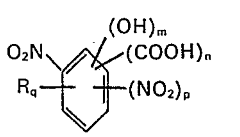

(I)

dans laquelle:

R, identique ou différent, représente un atome d'hydrogène ou d'halogène, un radical alcoyle contenant de 1 à 4 atomes de carbone, le groupe trifluorométhyle, sulfonamido ou méthylsulfonyle, et

m, n, p, q représentent chacun un nombre entier égal à 0 ou 1, q pouvant de plus être égal à 2,

par une base, à raison de 0,1 à 50% en moles par rapport au dérivé aromatique nitré brut, en solution dans un solvant organique inerte.

2. Procédé selon la revendication 1, caractérisé en ce que la base est minérale.

3. Procédé selon la revendication 2, caractérisé en ce que la base minérale est la soude, la potasse ou un carbonate alcalin.

4. Procédé selon la revendication 1, caractérisé en ce que la base est une amine primaire.

5. Procédé selon la revendication 1, caractérisé en ce que la base est ajoutée à raison de 0,1 à 30% en moles par rapport au produit brut.

6. Procédé selon la revendication 5, caractérisé en ce que la base est ajoutée à raison de 1 à 25% en moles par rapport au produit brut.

7. Procédé selon l'une des revendications 1 à 6, caractérisé en ce que le solvant est un alcanol contenant de 1 à 4 atomes de carbone.

8. Procédé selon la revendication 7, caractérisé en ce que le solvant est l'éthanol.

9. Procédé selon la revendication 1, caractérisé en ce que le solvant est l'éther de diisopropyle.

10. Procédé selon l'une des revendications 1 à 9, caractérisé en ce que le traitement est effectué à une température comprise entre la température ambiante et la température de reflux du mélange réactionnel.

11. Procédé selon l'une des revendications 1 à 10, caractérisé en ce qu'on traite un dérivé de formule I de la revendication 1, dans laquelle m et n sont différents ou sont tous deux égaux à 1.

12. Procédé selon la revendication 11, caractérisé en ce qu'on traite un phénol de formule:

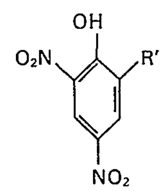

(II)

dans laquelle R' est un radical alcoyle contenant de 1 à 4 atomes de carbone.

13. Procédé selon la revendication 12, caractérisé en ce qu'on traite du dinoterbe.

## Patentansprüche

1. Verfahren zur Entfernung von Nitrosierungsmitteln aus nitrierten aromatischen Verbindungen, dadurch gekennzeichnet, dass man ein Rohprodukt behandelt, das nach seiner Produktion isoliert wurde und wesentlich von der nach der Nitrierungphase zurückbliebenden Nitriersäure freigemacht wurde, das wesentlich aus einer Verbindung der allgemeinen Formel I besteht:

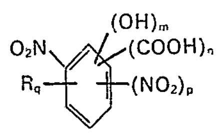

(I)

worin:

die Reste R, identisch oder verschieden, ein Wasserstoff oder Halogenatom, ein 1 bis 4 Kohlenstoffatomen enthaltende Alkylradikal, oder eine Trifluoromethyl-, Sulfonamide- oder Methylsulfonylgruppe, darstellen, und

jedes der m, n, p und q, eine ganze Zahl von 0 oder 1 darstellt mit der weitere Möglichkeit für q gleich 2 zu sein,

mit einer Base, in einer Menge von 0,1 bis 50 Mol-% in bezug auf das nitrierte aromatische Rohprodukt, in einer Lösung in einem inerten organischen Lösungsmittel behandelt wird.

2. Verfahren nach Anspruch 1, dadurch gekennzeichnet, dass die Base eine anorganische Base ist.

3. Verfahren nach Anspruch 2, dadurch gekennzeichnet, dass die anorganische Base Natriumhydroxyd, Kaliumhydroxyd oder Alkalikarbonat ist.

4. Verfahren nach Anspruch 1, dadurch gekennzeichnet, dass die Base in einer Menge von 0,1 bis 30 Mol-%, in bezug auf das Rohprodukt, eingesetzt wird.

6. Verfahren nach Anspruch 5, dadurch gekennzeichnet, dass die Base in einer Menge von 1 bis 25 Mol-%, in bezug auf das Rohprodukt, eingesetzt wird.

7. Verfahren nach Anspruch 1 bis 6, dadurch gekennzeichnet, dass das Lösungsmittel ein 1 bis 4 Kohlenstoffatomen enthaltende Alkanol ist.

8. Verfahren nach Anspruch 7, dadurch gekennzeichnet, dass das Lösungsmittel Äthanol ist.

9. Verfahren nach Anspruch 1, dadurch gekennzeichnet, dass das Lösungsmittel Diisopropyläther ist.

10. Verfahren nach einem der Ansprüche 1 bis 9, dadurch gekennzeichnet, dass die Behandlung bei einer Temperatur zwischen Raumtemperatur und Rückflusstemperatur des Reaktionsgemisches durchgeführt wird.

11. Verfahren nach einem der Ansprüche 1 bis 10, dadurch gekennzeichnet, dass die Behandlung einer Verbindung der allgemeinen Formel I durchgeführt wird worin m und n verschieden oder beide gleich 1 sind.

12. Verfahren nach Anspruch 11, dadurch gekennzeichnet, dass die Behandlung auf ein Phenol der allgemeinen Formel:

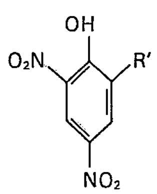

(II)

durchgeführt wird, worin R′ ein 1 bis 4 Kohlenstoffatomen enthaltende Alkylradikal ist.

13. Verfahren nach Anspruch 11, dadurch gekennzeichnet, dass die Behandlung auf Dinoterb durchgeführt wird.

## Claims

1. A process for removing nitrosating agents from aromatic nitro derivatives, characterized in that a crude product is treated which has been isolated after its fabrication and substantially made free from residual nitric acid originated from the nitration phase, consisting essentially of a compound of the general formula:

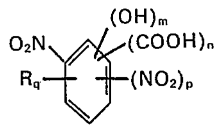

(I)

in which:

– the symbols R, which are identical or different, represent a hydrogen or halogen atom, an alkyl radical containing from 1 to 4 carbon atoms or the trifluoromethyl, sulphonamido or methylsulphonyl group, and

– m, n, p and q each represent an integer equal to 0 or 1, it also being possible for q to be equal to 2, with a base at the rate of 0.1 to 50 mol% relative to the crude aromatic nitro derivative, in solution in an inert organic solvent.

2. The process according to Claim 1, wherein the base is an inorganic base.

3. The process according to Claim 2, wherein the inorganic base is sodium hydroxide, potassium hydroxide or an alkali metal carbonate.

4. The process according to Claim 1, wherein the base is a primary amine.

5. The process according to Claim 1, wherein the base is added in an amount of 0.1 to 30 mol%, relative to the crude product.

6. The process according to Claim 5, wherein the base is added in an amount of 1 to 25 mol%, relative to the crude product.

7. The process according to one of Claims 1 to 6, wherein the solvent is an alkanol containing from 1 to 4 carbon atoms.

8. The process according to Claim 7, wherein the solvent is ethanol.

9. The process according to Claim 1, wherein the solvent is diisopropyl ether.

10. The process according to one of Claims 1 to 9, wherein the treatment is carried out at a temperature of between ambient temperature and the reflux temperature of the reaction mixture.

11. The process according to one of Claims 1 to 10, wherein the treatment is carried out on a derivative of the formula I, of Claim 1, in which m and n are different or are both equal to 1.

12. The process according to Claim 11, wherein the treatment is carried out on a phenol of the formula:

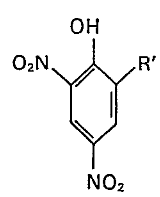

(II)

in which R′ is an alkyl radical containing from 1 to 4 carbon atoms.

13. The process according to Claim 12, wherein the treatment is carried out on dinoterb.